# EUROPEAN PATENT APPLICATION

(11) **EP 3 469 988 A1**
(43) Date of publication of application: **17.04.2019**
(21) Application number: 17195988.5
(22) Date of filing: 11.10.2017
(51) Int. Cl.: A61B 6/02, A61B 6/06, A61B 6/00, G21K 1/02

(54) **APPARATUS AND METHODS FOR IMAGING AN OBJECT BY CONE BEAM TOMOGRAPHY**

(71) Applicant: Ion Beam Applications S.A., 1348 Louvain-la-Neuve (BE)
(72) Inventor: Debatty, Alexandre, 1348 Louvain-la-Neuve (BE)
(74) Representative: ABYOO SPRL

(57) **Abstract**

The invention concerns an imaging apparatus (1) comprising a radiation detector (20), a radiation source (10) and an anti-scattering element (30), all rotatably mounted about a first rotation axis. The anti-scattering element (30) is a solid having the shape of a three-dimensional spiral-shaped or spirangle-shaped solid, with the wall of the solid orientated towards the focal point (12) of the radiation source. The anti-scattering element (30) allows reducing the signal-to-noise ratio of scattered radiations received by the radiation detector (20).

## Description

### Field of the invention

The invention relates to a cone beam tomography apparatus for imaging an object, said apparatus comprising an anti-scattering element.

The invention also relates to a method for imaging an object by using the cone beam tomography apparatus of the invention.

### Description of prior art

Imaging is an important diagnostic tool to the clinical assessment of patients. Numerous efforts have been made toward three-dimensional (3D) radiographic imaging. The introduction of cone beam tomography (CBT), or cone beam computed tomography (CBCT) dedicated to imaging a specific region of a patient, like a tumor, allows the reconstruction of 3D images of the imaged region. 3D-images are then used in radiography guidance. In CBCT, imaging is accomplished by using a rotating gantry with an x-ray source and a detector. A cone-shaped source of ionizing radiation is directed through an area of interest. During the rotation of the gantry, the radiation source and the detector circle about a region of interest to be scanned. A plurality of sequential planar projection images of the field of view is acquired and then computed by software for reconstructing a 3D image of the region of interest. Compared to traditional 2D approach, the cone beam computed tomography is able to achieve 3D images in a much shorter time, while minimizing exposure to radiations. CBCT is being increasingly used in modern radiation therapy for patient setup and adaptive replanning.

A significant limitation of existing cone beam imaging techniques occurs when the region of interest is larger than the field of view (FOV) of the detector. When the FOV of the detector is limited, it may be necessary to obtain a larger field size, to enhance the overall quality of the reconstructed 3D image. In US patent 7108421, an imaging apparatus with a translating detector and a cone beam positioner is described. By translating the detector to multiple positions, and obtaining images at these positions, a large FOV image may be acquired using a single detector and a single beam source.

Another significant limitation of CBCT is due to the large amount of radiation illumination directed through the area of interest. Scattering of radiation becomes a serious problem and is considered as one of the main limitations of CBCT image quality. Scatter causes severe distortions and contrast loss in the reconstructed images. Scattered radiation introduces two artifacts: reduction of the signal to noise ratio since the scattered photons contribute only to the noise while not carrying usable information on the imaged region of interest; and artifacts arise in the reconstructed image.

Many scatter correction methods have been proposed in the literature. To reduce the scattering effect, one common solution is to use scatter correction algorithms. But use of these algorithms is not possible in all and every situations, because none of them is able to correct scatter effect in all practical situations.

Another solution is to use anti-scattering elements. To reduce scattered radiation, an anti-scattering element is placed behind the area of interest to be imaged relatively to the radiation source, and in front of the detector. The anti-scattering element is designed to lower the scattered radiations that reach the detector, to enhance the overall quality of the 3D reconstructed image by reducing the signal-to-noise ratio. As an example, an anti-scatter grid is disclosed in US Pat. Appl. 15/327909. The grid comprises a series of modular units juxtaposed with each other as to form a series of focused channels for the passage of the focused imaging x-rays. A main issue with such anti-scatter grid is the existence of a variable time-shadowed/time-illuminated ratio for the various pixels present on the detector. Indeed, when an anti-scatter element is placed above the detector, it creates a shadow on the detector, and a portion of non-scattered radiation is unable to reach the detector. Due to the loss of useful signal, the overall quality of the image may be reduced, and the signal-to-noise ratio isn't fully satisfactory.

Despites the use of algorithms and/or of the current anti-scatter element, there is a need for improved quality of the reconstructed images in order to meet the very high performance standards required in medicinal field, and generate high quality and artifact-free images. There is also a need for generating high quality and artifact-free images in an extended field of view, with reduced scattered radiations.

### Summary of the invention

To solve at least partially the problems of the state of the art, it is an object of the invention to provide a cone beam tomography apparatus for imaging an object wherein the scattered radiations reaching the radiation detector are at least reduced. It is also an object of the invention to provide a cone beam tomography apparatus for imaging an object wherein an anti-scattering element may be used effectively in different fields of view. It is also an object of the invention to provide a cone beam tomography apparatus for imaging an object wherein an anti-scattering element is designed to avoid the problems related to the shadowed-time/illumination-time for each pixel on the detector. It is also an object of the invention to provide a cone beam tomography apparatus for imaging an object wherein the signal-to-noise ratio between non-scattered radiations and scattered radiations is enhanced.

The invention is defined by the independent claims. The dependent claims define advantageous embodiments.

According to the invention, there is provided a cone beam tomography apparatus for imaging an object, said apparatus comprising a radiation source configured to emit a conic radiation field having an optical axis A₁, a radiation detector mounted opposite to the radiation source to receive said conic radiation field , and an anti-scattering element disposed between the radiation source and the radiation detector and in front of the radiation detector, the apparatus being rotatably mounted about a first rotation axis R₁, said rotation axis R₁ being perpendicular to the optical axis A₁ and being located between the radiation source and the anti-scattering element. The apparatus is characterized in that the anti-scattering element comprises a first wall whose shape corresponds to a transversal section between a first and a second surface of a solid obtained by a first straight rod having one extremity kept at a focal point of the radiation source and an opposite extremity moved along a first planar spiral or a first n₁-segmented planar spirangle, with 2 < n₁ < ∞, which is perpendicular to A₁, and whose center point is on the optical axis A₁. The apparatus is also characterized in that the anti-scattering element is rotatably mounted about the axis A₁.

Indeed, the anti-scattering element with its spiral or spirangle shape and its wall orientated towards the apex of the spiral or spirangle located at the focal point of the radiation source is able to prevent a large amount of scattered radiations to reach the detector, thereby reducing the noise, while the non-scattered radiations may reach the detector by passing between the wall of the anti-scattering element. Moreover, due to the rotation of the anti-scattering element, each pixel has a shadowed-time/illumination-time (or light/shadow time) more consistent with the overall shadowed-time/illumination-time of each pixel of the detector, thereby enhancing the overall quality of the reconstructed image. With such anti-scattering element, the use of post-treatment analysis and correction may be reduced. It is also possible to have a constant for each pixel of the detector between the illumination time (when non-scattered radiations may reach the detector) and the shadowed time (when non-scattered radiations are unable to reach the detector because they hit the upper surface of the anti-scattering element's wall), thereby enhancing the overall quality of the image, and/or reducing post-treatments analysis or corrections.

Preferably, the apparatus according to the invention comprises an anti-scattering element, wherein the pitch of the first spiral or of the first n₁-segmented spirangle decreases with the radius of the spiral or of the n₁-segmented spirangle.

Indeed, with such a shape, the time-shadowed/time-illuminated ratio of each pixel located on the detector is more even distributed, and therefore the images obtained need less post-treatment, like the usual algorithms used to enhance the quality of the final reconstructed image. It is also possible to have a constant time-shadowed/time-illuminated ratio for each pixel on the detector.

More preferably, the apparatus according to the invention has the anti-scattering element mounted in translation in a first plane perpendicular to the optical axis A₁, the anti-scattering element being furthermore rotatably mounted about a second rotation axis R₂, said second rotation axis R₂ being perpendicular to the optical axis A₁ and parallel to the first plane, and passing through the anti-scattering element.

An apparatus according to this preferred embodiment allows the imaging of an object in a large field of view operation, while the anti-scattering element is still able to filter scattered radiations, thereby enhancing the overall quality of images scanned in a large field of view operation. According to this embodiment, the apparatus is able to image an object larger than the native field of view of the apparatus, due to the size limitation of the radiation detector, and/or due to the size of the cone beam emitted by the radiation source.

In a more preferred embodiment, and particularly when the detector has a shape of a flat panel, the radiation detector is mounted in translation in a second plane parallel to the first plane. Therefore, a small radiation detector may be used even in large field of view imaging.

### Short description of the drawings

These and further aspects of the invention will be explained in greater detail by way of examples and with reference to the accompanying drawings in which:
- Fig.1: shows a schematic diagram of a cone beam apparatus according to one embodiment of the invention.
- Fig.2: shows schematic diagrams of a cone beam apparatus according to another embodiment of the invention when the apparatus is imaging with a large field of view, with two different anti-scattering elements according to the invention.
- Fig.3: shows a side section, an isometric projection, and a top view of an anti-scattering element according to a fist embodiment of the invention.
- Fig.4: shows a schematic view of the anti-scattering element according to the first embodiment of the invention.
- Fig.5: shows a schematic isometric projection of the anti-scattering element according to the first embodiment of the invention.
- Fig.6: shows a schematic top view of an anti-scattering element according to the first embodiment of the invention.
- Fig.7: shows a schematic isometric projection of an anti-scattering element according to a second embodiment of the invention.
- Fig.8: shows a schematic top view of the anti-scattering element according to the second embodiment of the invention.
- Fig.9: shows a schematic isometric projection of an anti-scattering element according to a third embodiment of the invention.
- Fig.10: shows a schematic top view of the anti-scattering element according to the third embodiment of the invention.
- Fig.11: shows a schematic isometric projection of an anti-scattering element according to a fourth embodiment of the invention.
- Fig.12: shows a schematic top view of the anti-scattering element according to the fourth embodiment of the invention.

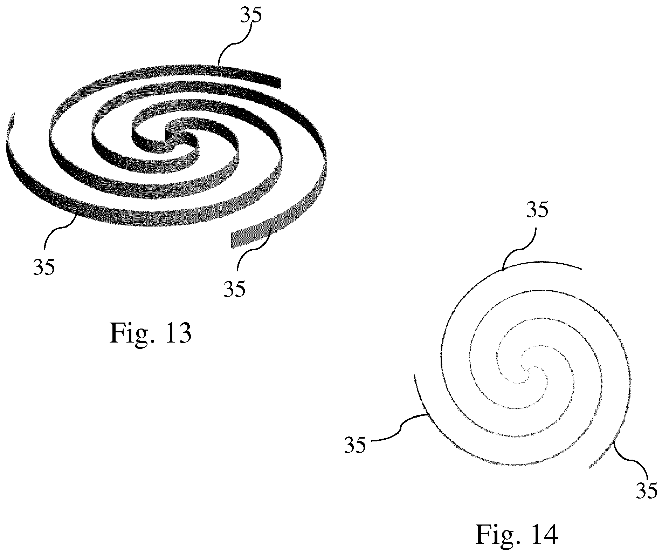

- Fig.13: shows a schematic isometric projection of an anti-scattering element according to a fifth embodiment of the invention.
- Fig.14: shows a schematic top view of the anti-scattering element according to the fifth embodiment of the invention.

The drawings of the figures are neither drawn to scale nor proportioned. Generally, similar or identical components are denoted by the same reference numerals in the figures.

### Detailed description of embodiments of the invention

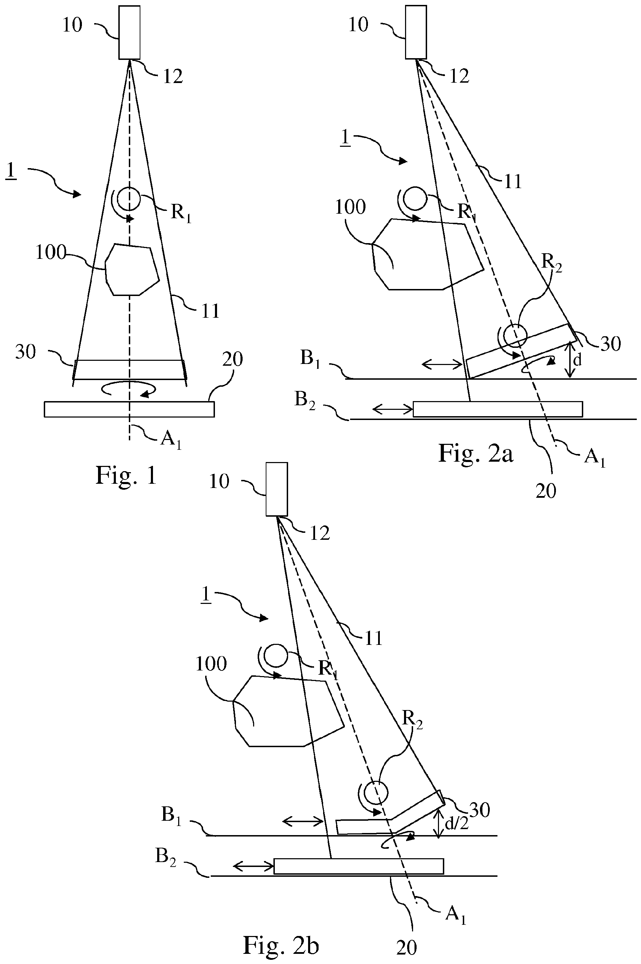

A cone beam apparatus (1) according to a first embodiment of the invention for imaging an object (100) is schematically represented on Fig.1.

Such system is typically included within a gantry, which is not represented on the various Fig. A gantry is well known in the art, and generally comprises a circular housing with a central opening in which a patient or an object (100) to be imaged may be placed on a support, typically on a chair or a flat support. The apparatus comprises a radiation source (10). The radiation source (10) is generally an x-ray source. In a preferred embodiment of the invention, the radiation source (10) is an X-ray radiation source. The x-ray source may be an anode pulsed x-ray source for example, but other x-ray sources may be contemplated. The radiation source is configured to emit a conic radiation field (11), typically x-rays radiations, towards a radiation detector (20). The conic radiation field may be a right circular cone. The conic radiation field (11) has an optical axis A₁. The optical axis A₁ may be defined as the axis of a right circular cone when the conic radiation field is a right circular cone. A₁ passes through a focal point (12) of the radiation source (10). The focal point (12) could be defined as a point of sources of the radiations emitted by the radiation source (10).

The apparatus also comprises a radiation detector (20). A radiation detector is able to record radiation emitted by the radiation source (10), like x-rays photons. The radiation detector is mounted in the apparatus so that the radiation detector (20) receives radiations emitted by the source. Therefore, the radiation detector (20) is mounted opposite to the radiation source (10) regarding a rotation axis R₁.

In a particular embodiment of the invention, the radiation detector (20) is a flat-panel detector. The radiation detector (20) may be for example a scintillation screen, image intensifiers, or charge-coupled device (CCD) detectors. All these radiation detectors are well known in the art. In a preferred embodiment of the invention, the detector is composed of a pixel array of x-rays sensitive material, like hydrogenated amorphous silicon film transistors. X-rays may be detected by the indirect use of a scintillator, such as terbium-activated gadolinium oxysulfide or thallium-doped cesium iodide, which converts x-rays into visible light subsequently registered in a photo diode array. In a preferred embodiment, the radiation detector is a rectangular or square flat-panel. The size of one side may be comprised between 40 cm and 80 cm, while the size of the other size may be comprised between 40 and 80 cm. In a preferred embodiment, the flat panel is a 40 cm by 40 cm flat square.

The apparatus also comprises an anti-scattering element (30). As illustrated on Fig. 1, the anti-scattering (30) element is disposed between the radiation source (10) and the radiation detector (20), and in front of the radiation detector (20). In other words, it should be understood that the anti-scattering element (30) is disposed opposite to the radiation source (10) regarding the rotation axis R₁. By in front, it should be understood that the anti-scattering element (30) is disposed few centimeters in front of the radiation detector (20), for example, between 0,1 cm and 1 cm. The aim of the anti-scattering element (30) is to prevent scattered radiations to reach the radiation detector (20). Indeed, when the radiation emitted by the radiation source reaches the object to be imaged, some of the radiations are scattered by the object, and therefore do not follow their normal path (a movement in a straight direction from the radiation source towards the radiation detector). These scattered radiations contribute to a noise that lowers the overall quality of the imaging process. The anti-scattering element (30) is therefore able to prevent these scattered radiations to reach the radiation detector (20), while the non-scattered radiations are able to reach the radiation detector. Hence, the signal-to-noise ratio is greatly enhanced, improving the quality of the images, and/or lower the post-processing treatments of the signals detected by the radiation detector (20). The anti-scattering element may comprise an element with a high atomic number, like lead, titanium, or tungsten. In a preferred embodiment, the anti-scattering element in made in an alloy comprising an element with a high atomic number, like lead, titanium, or tungsten.

The apparatus is rotatably mounted about the first rotation axis R₁. In other words, the radiation source (10) is rotatably mounted about the first rotation axis R₁, the radiation detector (20) is rotatably mounted about the first rotation axis R₁, and the anti-scattering element (30) is rotatably mounted about the first rotation axis R₁. As illustrated on Fig.1, both the radiation detector (20) and the anti-scattering element (30) are mounted opposite to the radiation source (10) regarding the location of rotation axis R₁. In other words, the rotation axis R1 is located between the radiation source (10) and the anti-scattering element (30). Rotation axis R₁ is perpendicular to the optical axis A₁, and preferably crosses the optical axis A₁.

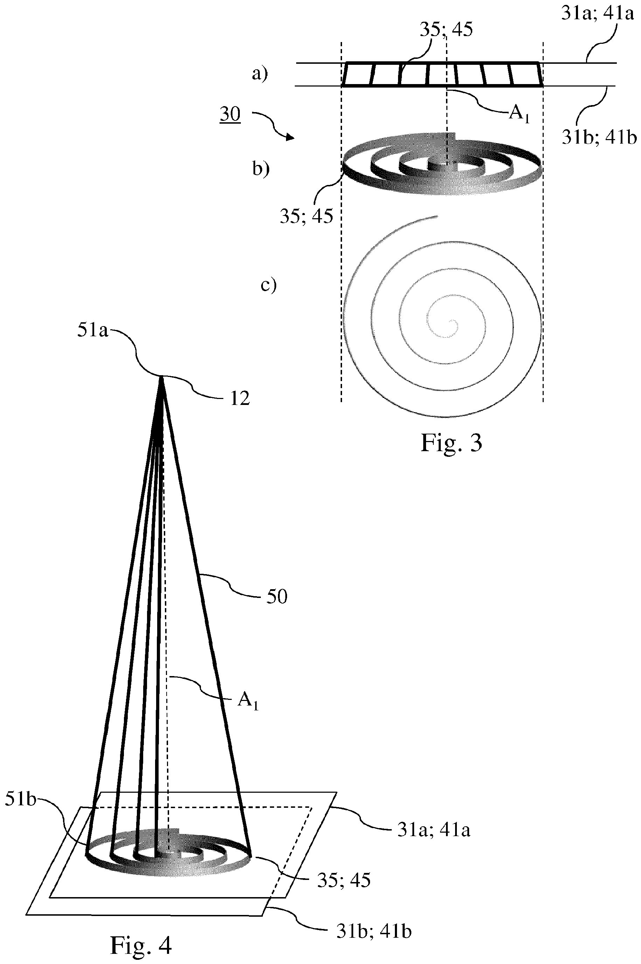

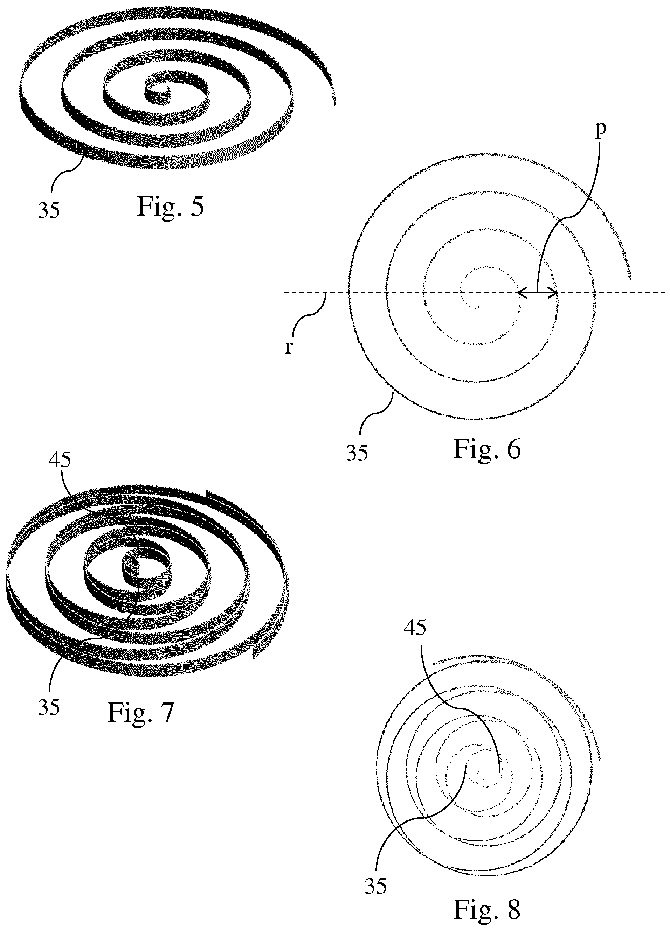

A first embodiment of an anti-scattering element (30) according to the invention is illustrated on Fig. 3. Fig. 3 comprises 3 different views of an anti-scattering element (30): a side section (a); an isometric projection (b) and a top view (c) of a single anti-scattering element (30). The anti-scattering element (30) comprises a fist wall (35). The shape of the wall (35) corresponds to a transversal section of a solid between a first surface (31 a) and a second surface (31 b). The solid is obtained by the movement of a rod segment, one extremity of the rod being kept at the focal point (12) of the radiation source (10), and an opposed extremity of the rod being moved along a first planar spiral or a first n₁ segmented planar spirangle, with 2 < n₁ < ∞. The first spiral or first spirangle is perpendicular to optical axis A₁. The center point of the spiral of spirangle is also located on the optical axis A₁. This is illustrated on Fig. 4, wherein the rod (50) is illustrated in bold lines at various positioning. As one can see, one extremity (51 a) is kept at focal point (12); while the other extremity (51 b) draws a spiral or a spirangle. The solid is then obtained by a section between two surfaces; a first surface (here represented as the upper surface) (31 a), and a second surface (here represented as the lower surface) (31 b). These surfaces are represented as flat surfaces, but other surface may be contemplated. In other words, the first wall has the shape of a three-dimensional solid section; said solid being created from a rod whose one extremity is fixed while its other extremity turns around optical axis A₁ at a constant or varying distance from the center point. This result in a three-dimensional spiral-shaped or spirangle-shaped solid, with the wall of the solid orientated towards the focal point (12) of the radiation source. This is illustrated on Fig.3a and Fig.4, wherein it is clear that wall's tangent converge towards the focal point (12). Since one of the extremities of the rod is fixed, helix and cylindrical coil are not included in this definition.
When the turning extremity of the rod turns around the center point at a constant distance, the anti-scattering element illustrated on Fig. 5 and 6 is obtained. In other words, the pitch (p) of the spiral or the pitch (p) of the spirangle is constant. The pitch may be defined as the radial winding pitch, or the radial distance between two points of the spiral of spirangle which are separated by one turn, as illustrated on Fig. 6.

In another embodiment of the invention, illustrated on Fig.7 and 8, the anti-scattering element may comprise at least 2 walls. The first wall (35) may be obtained according to any one of the embodiments described therein. The second wall (45) has a shape whose corresponds to a transversal section between a third surface (41 a) and a fourth surface (41 b) of a solid. In a similar fashion with the first wall (35), the second solid is obtained by a second straight rod: one of its extremities is kept at the focal point (12) of the radiation source, and another extremity is moved along a second planar spiral or second n₂ segmented planar spirangle, with 2 < n₂ < ∞. According to this embodiment, the first spiral or the first spirangle is left-handed, and the second spiral or second spirangle is right-handed. In other words, the movement of the moving extremity of the first rod is laevorotatory, and the movement of the moving extremity of the second rod is dextrorotatory (or rather the opposite). An anti-scattering element (30) according to this embodiment is sturdy, and may resist higher speed rotation that the single spiral embodiment. In a particular embodiment, n₁ = n₂. In a particular version of this embodiment, the first spiral or the first spirangle on one hand, and the second spiral or the second spirangle on the other hand, have the same pitch. Alternatively or complementarily, the first wall and the second wall may have the same thickness while their radius increases or decreases. In a more preferred version of this embodiment, the second wall is the image of the first wall in a mirror. In other words, the first surface (31 a) and the third surface (41 a) are the same; and the second surface (31 b) and the second surface (41 b) are the same. According to this embodiment, the first wall (35) and the second wall (45) share the same features, except that one is left-handed and the other one is right-handed.

Regarding the surfaces (31 a, 31 b, 41 a, 41 b, illustrated on Fig. 4), these surfaces limit the section of the solids. These surfaces could be defined as mathematical surfaces. The surfaces may be planes and perpendicular to optical axis A₁, thereby forming a solid frustum. In other embodiment, one surface may be plane, for example the first and third surfaces, who could also be defined as the upper surfaces, while the other surfaces are right circular conical surfaces. In a preferred embodiment, the apexes of the right circular conical surfaces are located on the optical axis A₁, and the axis of the conical surfaces is optical axis A₁. When the lower surface of the anti-scattering element (30) has the shape of a right circular conical surface, the apexes of the right circular conical surfaces being located on the optical axis A₁, the anti-scattering element may be tilted on itself. This embodiment is particularly advantageous when the apparatus is working in a large field of view. In a more preferred embodiment, all surfaces (31 a, 31 b, 41 a, 41 b) are right circular conical surfaces orientated opposite to the radiation source (10), with their apexes located on the optical axis A1, the axis of the right circular conical surfaces being optical axis A₁. Such embodiment is particularly advantageous in large field of view operations, when the anti-scattering element and the radiation detector are mounted in translation. This is for example illustrated on Fig.2b. It should be noted that other shapes are worth considering. For example, the surfaces may be concave or convex surfaces, regular or not, pyramidal surfaces, regular or not, the upper surfaces (31 a, 41 a) may have the same shape as the lower surface (31 b, 41 b), but the upper surface (31 a, 41 a) and the lower surfaces (31 b, 41 b) may have a different shape.

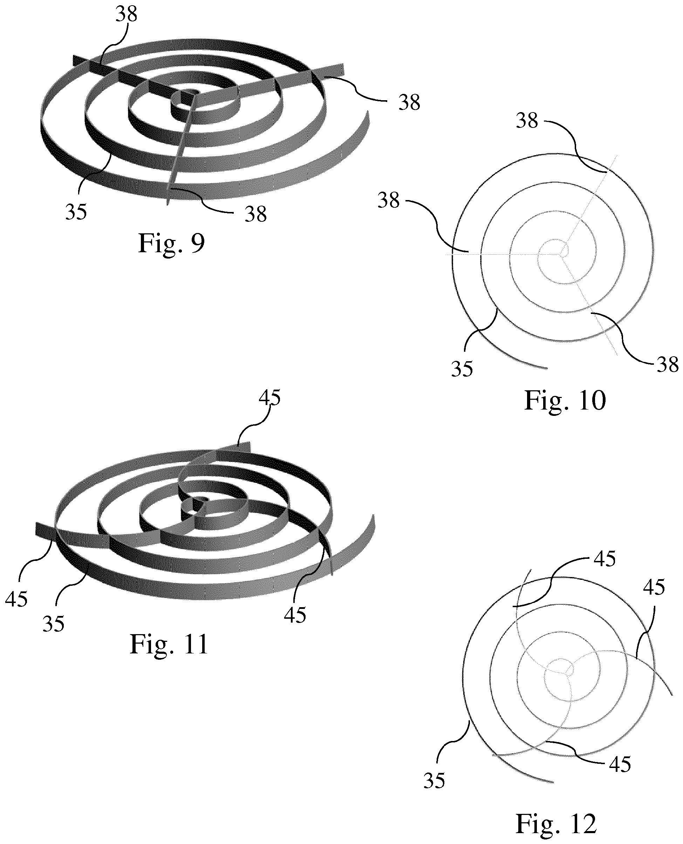

In a preferred embodiment of the invention, the anti-scattering element (30) may comprise a radial wall (38), or a plurality of radial walls (38). This is illustrated on Fig. 9 and 10. A radial wall (38) may be defined as a straight wall located at least partially on a radius of the spiral or the spirangle. With such radial wall (38), the anti-scattering element is more resistant and is less subject to deformation while the rotation speed increases. Hence, an anti-scattering element (30) according to this embodiment may be used at high-speed rotation. Alternatively, a radial wall (38) may be replaced by a diameter wall, or a plurality of diameter walls.

In an alternative embodiment of the invention, illustrated on Fig.11 and 12, the anti-scattering element (30) comprises a plurality of second walls (45), each sharing a same shape, but emerging from the center point of the first spiral or first spirangle at various winding angle. As an example, the anti-scattering apparatus may comprise 3 second walls (45), each having the same shape, and the same pitch, and the same center point, each spiral emerging from the center point with a winding angle of respectively 0 °, 120 ° and 240 °. Of course, a various number of second walls (45) may be incorporated into the anti-scattering element (30).

According to alternative embodiments, the turning extremity of the rod may turn about the center point at a variable distance. The turning extremity of the rod may turn about the center point with a decreasing pitch. In other words, the pitch of the spiral or the pitch of the spirangle decreases with the radius of the spiral of with the radius of the spirangle. The more the wall is distant from the center point, the more the distance between two consecutive portions of the wall located on a same radius are closer to each other. Such configuration may allow reducing the shadowed-time/illumination-time differences on the particular pixels of the radiation detector. With such configuration, the pixels located in a central portion and the pixels located on edge portions of the radiation detector (20) receive a more consistent amount of illumination during the radiation. In a more preferred embodiment, the pitch of the first spiral or the pitch of the first spirangle may decrease or increase with the radius. With a spiral or spirangle with a pitch according to this embodiment, the shadowed-time/illumination-time differences for all pixels of the radiation detector (20) may be even more reduced, thereby leading to a more even ratio of illumination time and shadow time on each pixel, enhancing the overall quality of the imaging process, and/or facilitating post-treatment of the image.

In another embodiment of the invention, the thickness of the first wall (35) increases with the radius of the spiral or of the spirangle. This embodiment allows a more consistent ratio of illuminated-time / shadowed-time for all the pixels of the radiation detector (20). The thickness of the wall may be comprised between 0.1 mm and 10 mm. In other words, the thickness of the rod used to shape the wall may be comprised between 0.1 mm and 10 mm. In a particular embodiment of the invention, the thickness of the wall increases at a constant factor with the radius. Alternatively, the thickness of the wall decreases at a constant factor with the radius.

The anti-scattering element (30) is rotatably mounted about the optical axis A₁. When the device is in use, the anti-scattering element (30) rotates about A₁, thereby reducing the amount of scattered radiation that reaches the radiation detector (20). Depending on the shape of the anti-scattering element (30) approximatively 80% of the radiations scattered by the object to be imaged are prevented from reaching the surface of the radiation detector (20), more preferably approximatively 90% of the radiations scattered by the object to be imaged are prevented from reaching the surface of the radiation detector (20), and still more preferably, approximatively 95% of the radiations scattered by the object to be imaged are prevented from reaching the surface of the radiation detector (20). In some embodiments, 100% of the radiations scattered by the object to be imaged are prevented from reaching the surface of the radiation. To rotate the anti-scattering element (30) about A₁, the anti-scattering element may be hooped by a ring, the ring being connected to rotational means configured to rotate the anti-scattering element (30) about R₁.

In a more particular embodiment of the invention, the pitch of the spiral or the pitch of the spirangle on one hand, and the thickness of the wall on the other end are variables. The pitch of the spiral or the pitch of the spirangle may decrease with the radius, while the thickness of the wall may increase with the radius. This embodiment may reduce the ratio differences of illuminated-time / shadowed-time for all or particular pixels of the radiation detector (20).

In a complementary or alternative embodiment of the invention, the first wall, and the case being the second wall, or the case being any wall, has a height comprised between 0,5 cm and 2 cm. The height of wall(s) may be constant, or may increase of decrease with the radius of the spiral or the radius of the spirangle. The height of the wall may be defined as the distance between a base of the wall, in contact with the lower surface (31 b, 41 b), and a top of the wall, in contact with the upper surface (31 a, 41 a). It should be noted that the height may increase with the radius, decrease with the radius, or first increase and then decrease with radius, or first decrease and then increase with the radius.

In a second general embodiment of the invention, illustrated on Fig.2b, the apparatus (2) is adapted to perform imaging of an object with a large field of view. To this end, any anti-scattering element (30) described earlier in this specification is mounted in translation in a first plane (B₁). The first plane B₁ The first plane B₁ is perpendicular to the optical axis A₁. In this embodiment, the anti-scattering element (30) is also rotatably mounted about a second rotation axis R₂. The rotation axis R₂ is perpendicular to the optical axis A₁. The rotation axis R₂ is also perpendicular to the translation direction of the scattering element. The rotation axis R₂ passes through the scattering element (30). As an example, the anti-scattering element (30) may be hooped by a ring, the ring being connected to rotational means configured to rotate the anti-scattering element about R₂. According to this embodiment, the anti-scattering element is able to be translated on a perpendicular plane to optical axis A₁, and then rotate about R₂, so that the anti-scattering element is able to rotate again around the optical axis A₁, as illustrated on Fig. 2b. With such embodiment, the size of the field of view of the apparatus is enhanced, allowing larger object to be imaged. It should be noted that according to this embodiment, an anti-scattering element (30) with the lower surfaces (31 b, and 41 b the case being) being right circular conical surfaces orientated opposite to the radiation source (10), with their apexes located on the optical axis A1, the axis of the right circular conical surfaces being optical axis A₁, allows the rotation axis R₂ to be located near the upper surface of the radiation detector (20). Thereby, the lower surface of the anti-scattering element is not too distant from the radiation detector, as clearly illustrated on fig.2b. With such "folded" anti-scattering element, at least a part of the lower surface of the anti-scattering element may be kept at a close distance from the upper surface of the radiation detector. As illustrated on Fig.2a and Fig.2b, the maximum distance d between the lower surface of a flat anti-scattering element and the upper surface of the radiation detector may be divided by two with a "folded" anti-scattering element (Fig.2b) and after rotation of the anti-scattering element about R₂. One other advantageous effect is that the overall space occupied by the combination of the detector and the anti-scattering element may be reduced in large FOV imaging. Therefore, the space available for the patient is larger.

In a complementary embodiment, the radiation detector (10) is mounted in translation in a plane B₂, B₂ being parallel to plane B₁. This allows the apparatus to perform large field of view imaging, with a size-reduced radiation detector (20). Such embodiment allows the apparatus to work in large field of view imaging, while the size of the scattering element (30) and the size of the radiation detector (20) are reduced, lowering the overall size of the apparatus.

In a complementary embodiment, the radiation source (10) may also comprise an orientation means able to tilt or translate the radiation source according to the translations of the anti-scattering element (30) and the case being the radiation detector (20). This allows the radiation source to emit the entire conic radiation field towards the anti-scattering element and the case being the radiation detector as illustrated on Fig.2b. Alternatively, or complementarily, the radiation source (10) may emit a large cone beam, while the anti-scattering element (30) and, the case being, the radiation detector translates during the imaging process.

The present disclosure also concerns a method for imaging an object. The first step of the method comprises the furniture of an apparatus according to any one already disclosed in this specification.

The method also comprises the following steps:
b) positioning an object to be imaged within the conic radiation field;
c) rotating the apparatus about the rotation axis R₁ over at least 360° and rotating the anti-scattering element about axis A₁;
d) irradiating the object to be imaged by activating the radiation source;
e) detecting radiation at the detector during the irradiation step d);
f) creating a three-dimensional image of the object by using the detected radiation at the radiation detector during the detection step e).

The method could furthermore comprise the following steps when the apparatus is working in large field of view imaging:
- the radiation detector (10) and the anti-scattering element (30) are translated perpendicularly to the optical axis A₁ and in a common direction,
- the radiation source (20) is tilted for emitting the radiation field (11) towards a new position of the radiation detector (20) and of the anti-scattering element (30).

A further step may be present: the anti-scattering element (30) may be rotated around the rotation axis R₂ so that the anti-scattering element is able to rotate about optical axis A₁.

The present invention has been described in terms of specific embodiments, which are illustrative of the invention and not to be construed as limiting. More generally, it will be appreciated by persons skilled in the art that the present invention is not limited by what has been particularly shown and/or described hereinabove.
Reference numerals in the claims do not limit their protective scope.
Use of the verbs "to comprise", "to include", "to be composed of", or any other variant, as well as their respective conjugations, does not exclude the presence of elements other than those stated.
Use of the article "a", "an" or "the" preceding an element does not exclude the presence of a plurality of such elements.

The invention may also be described as follows:
The invention concerns an imaging apparatus (1) comprising a radiation detector (20), a radiation source (10) and an anti-scattering element (30), all rotatably mounted about a first rotation axis. The anti-scattering element (30) is a solid having the shape of a three-dimensional spiral-shaped or spirangle-shaped solid, with the wall of the solid orientated towards the focal point (12) of the radiation source. The anti-scattering element (30) allows reducing the signal-to-noise ratio of scattered radiations received by the radiation detector (20).

## Claims

1. A cone beam tomography apparatus (1) for imaging an object, said apparatus (1) comprising a radiation source (10) configured to emit a conic radiation field (11) having an optical axis A₁, a radiation detector (20) mounted opposite to the radiation source (10) to receive said conic radiation field (11), and an anti-scattering element (30) disposed between the radiation source (10) and the radiation detector (20) and in front of the radiation detector (20), the apparatus being rotatably mounted about a first rotation axis R₁, said rotation axis R₁ being perpendicular to the optical axis A₁ and being located between the radiation source (10) and the anti-scattering element (30),
**characterized in that**
- the anti-scattering element (30) comprises a first wall (35) whose shape corresponds to a transversal section between a first (31 a) and a second surface (31 b) of a solid obtained by a first straight rod having one extremity kept at a focal point (12) of the radiation source (10) and an opposite extremity moved along a first planar spiral or a first n₁-segmented planar spirangle, with 2 < n₁ < ∞, which is perpendicular to optical axis A₁, and whose center point is on the optical axis A₁;
- the anti-scattering element (30) is rotatably mounted about the axis A₁.

2. The apparatus according to claim 1, wherein the pitch of the first spiral or of the first n₁-segmented spirangle decreases with the radius of the spiral or of the n₁-segmented spirangle.

3. The apparatus according to any one of the previous claims, wherein a thickness of the first wall increases with the radius of the spiral or of the n₁-segmented spirangle.

4. The apparatus according to any one of the previous claims, wherein the anti-scattering element (30) furthermore comprises a second wall (45), whose shape corresponds to a transversal section between a third (41 a) and a fourth surface (11 b) of a solid obtained by a second straight rod having one extremity kept at a focal point (12) of the radiation source (10) and an opposite extremity moved along a second planar spiral or a second n₂-segmented planar spirangle, with 2 < n₂ < ∞, which is perpendicular to A₁, and whose center point is on the optical axis A₁; and wherein the first spiral or first spirangle is left-handed and the second spiral or second spirangle is right-handed.

5. The apparatus according to claim 4, wherein the first surface (31 a) and the third surface (41 a) are the same, and wherein the second surface (31 b) and the fourth surface (41 b) are the same.

6. The apparatus according to any one of the previous claims, wherein the anti-scattering element (30) is mounted in translation in a first plane perpendicular to the optical axis A₁, the anti-scattering element (30) being furthermore rotatably mounted about a second rotation axis R₂, said second rotation axis R₂ being perpendicular to the optical axis A₁, perpendicular to the translation direction of the scattering element (30), and passing through the anti-scattering element.

7. The apparatus according to claim 6, wherein the radiation detector (10) is mounted in translation in a second plane parallel to the first plane.

8. An apparatus according to any one of the previous claims, wherein the first (31 a) and the second surface (31 b) are two parallels planes, preferentially perpendicular to the axis A₁.

9. An apparatus according to any one of the previous claims, wherein the first surface (31 a) and the second surface (31 b) are right circular conical surfaces, with their apexes orientated opposite to the radiation source (10), said apexes being located on the optical axis A1, and the axis of the conical surfaces is optical axis A₁.

10. An apparatus according to any one of the previous claims, wherein a height of the first wall (35) is comprised between 0,5 cm and 2 cm.

11. An apparatus according to any one of the previous claims, wherein the straight rod has a thickness comprised between 0.1 mm and 10 mm.

12. A method for imaging an object, said method comprising the following steps:
a) furnishing an apparatus according to any one of claims 1 to 11,
b) positioning an object to be imaged within the conic radiation field;
c) rotating the apparatus about the rotation axis R₁ over at least 360° and rotating the anti-scattering element about axis A₁;
d) irradiating the object to be imaged by activating the radiation source;
e) detecting radiation at the detector during the irradiation step d);
f) creating a three-dimensional image of the object by using the detected radiation at the radiation detector during the detection step e).

13. The method according to claim 12, wherein the apparatus is an apparatus according to claim 6 or claim 7,
and wherein the radiation detector (10) and the anti-scattering element (30) are translated perpendicularly to the optical axis A₁ and in a common direction,
and wherein the radiation source is tilted for emitting the radiation field (11) towards a new position of the radiation detector (20) and of the anti-scattering element(30)

14. The method according to claim 13, wherein the anti-scattering element (30) is rotated around the rotation axis R₂ so that the anti-scattering element is able again to rotate about optical axis A₁.
